# EUROPEAN PATENT APPLICATION

(11) **EP 3 995 602 A1**
(43) Date of publication of application: **11.05.2022**
(21) Application number: 20794976.9
(22) Date of filing: 22.04.2020
(51) Int. Cl.: C23C 14/34, C23C 14/56, C23C 14/58

(54) **EQUIPMENT AND METHOD FOR DEPOSITING SPRAYED MATERIALS ON PARTICULATE MATERIALS**

(30) Priority: 25.04.2019 BR 102019008353
(71) Applicant: União Brasileira de Educacão e Assistência, 90619-900, Porto Alegre, RS (BR)
(72) Inventor: FEIL, Adriano Friedrich, 90830-244 Porto Alegre (BR); EBERHARDT, Dario, 94931-023 Cachoeirinha (BR); DA SILVA, Pedro Migowski, 90420-030 Porto Alegre (BR); SOAVE, Claudio, 95700-000 Bento Gonçalves (BR)
(74) Representative: Comoglio, Elena
(86) International application number: PCT/BR2020/050135
(87) International publication number: WO 2020/215139

(57) **Abstract**

The present invention describes equipment and a method that are capable of carrying out the coating of metallic material on particulate materials through cathodic spraying (sputtering). The main advantages of the invention are sterile and semi-continuous production, precision in the thickness of the covered coat, as well as the reduction of occupational costs, since it is a semi-automated process.

The present invention is especially for application in the manufacture of raw materials to be used in the medical field.

## Description

### Field of the Invention

The present invention describes equipment and a method that are capable of carrying out the deposition of particulate materials through cathodic pulverization (sputtering) in a sterile manner and in semi-continuous production. More specifically, the invention describes equipment and a method for the deposition (coating) of particulate materials, especially polymethyl methacrylate (PMMA).

The equipment and method constituting the object of this invention have direct application in the medical field but are not limited thereto, with possible applications in the chemical industry, the aerospace industry, the metal working industry, the food industry, among others.

The present invention relates to the fields of engineering, physics, chemistry, and medicine, especially aesthetic medicine and plastic surgery.

### Background of the Invention

Some noteworthy processes that are employed by the industry when carrying out the deposition of materials include but are not limited to the electrolytic bath, laser ablation, thermal evaporation, electron beam, and sputtering.

The electrolytic bath, also known as electroplating, refers to a process for coating conductive or non-conductive materials with metals from a solution containing ions of these metals.

Laser ablation is characterized by a physical process in which a controlled laser pulse is aimed at a target and removes clusters of that target by breaking bonds through momentum transfer. Clusters ejected from the target are deposited on a substrate.

Thermal evaporation is the heating of a target material to its sublimation point using electrical resistances. At that point, the material begins to evaporate. The evaporated material is deposited on a substrate.

Similarly to the thermal evaporation process, the electron beam consists of heating a target material to its sublimation point using a beam of electrons that is aimed at it. The evaporated material is deposited on a substrate.

In sputtering, the substrate (i.e., the material to be deposited) is bombarded with the target (deposition material) by means of guns inside a vacuum chamber.

Sputtering is a process that has advantages over others, such as: less environmental and occupational impact, less operating space and, above all, a longer-lasting bond between materials, resulting in a product of incomparable quality.

In a search of the prior art in national and international databases, some documents were found which relate to methods for depositing particulate materials through sputtering.

Document number RU2477763-C1, entitled "*Method for obtaining polymer nanocomposite material*," with application in the area of metallurgy, describes a method for depositing powdered polymeric materials in a vacuum chamber. However, the aforementioned method comprises parameters and steps which differ from the claimed subject matter, and it does not teach an inert/aseptic manufacturing method.

Document number CN108219537-A, entitled "*Polyhedral pigment and preparation method thereof*, "describes a technology in the field of pigments. Such pigment was coated (deposited) with aluminum using the sputtering process. The method describes temperature control as a function of time and also employs inert gases such as nitrogen and argon. However, the description of that method does not teach aseptic manufacturing.

Document number US8968699, entitled, "Switchable nano-vehicle delivery systems and methods for making and using them," discloses powdered polymeric materials, including the materials polymethyl methacrylate, coated (through deposition) with metallic materials. However, the method for depositing such metals on polymeric powder material through sputtering is not described.

Therefore, no document from the prior art has anticipated the teachings described in the present document.

### Summary of the Invention

In a first aspect, the present invention provides equipment for sputtering metals onto particulate materials.

It is another object of the present invention to provide sputtering equipment comprising an inlet antechamber, a deposition chamber, and an outlet antechamber.

It is a further object of the present invention to provide a deposition chamber comprising at least one spray gun.

It is also an additional object to provide a deposition chamber comprising an agitator.

It is yet another object of the invention to provide an automated method for opening the hermetic container and conveyance from the inlet antechamber to the deposition chamber through the use of a pressure difference between the chambers.

The object of the present invention is to provide an automated method for conveying the particulate material from the deposition chamber to the outlet antechamber and hermetically closing the container.

It is also an object of the present invention to provide the method for depositing particulate materials in a sterile manner and in semi-continuous production.

It is an object of the present invention to provide for deposition of particulate materials through sputtering;

It is an object of the present invention to provide a method comprising:
a) fractionating the particulate materials;
b) sterilizing the particulate materials;
c) drying the particulate materials;
d) loading the particulate materials into the deposition antechamber;
e) coating the particulate materials in the deposition chamber;
f) storing, checking, and sterilizing the particulate materials.

It is a special object of the present invention that the particulate materials comprise polymethyl methacrylate (PMMA), preferably of micrometric size;

It is also an object of this invention that the powdered materials comprise the metals Ti, Ag, Au and combinations thereof; however, they are not limited to these and may also be Al, Si, Ga, Ge, V, Cr, Mn, Fe, Co, Ni, Cu, Zn , Zr, Nb, Mo, Ru, Rh, Pd, Cd, In, Sn, Sb, Hf, Ta, W, Re, Os, Ir, Pt, TI, Pb, Bi and combinations thereof, it also being possible for the respective oxides, nitrides, carbides, and combinations thereof to be used.

A further object of the present invention is a semi-continuous production process.

It is also object of the present invention to provide for the checking of deposition parameters such as the opening and closing of valves, gas injection, positioning of the hermetic cup, control and measurement of vacuum, and control and measurement of pressure by automation.

### Brief Description of the Figures

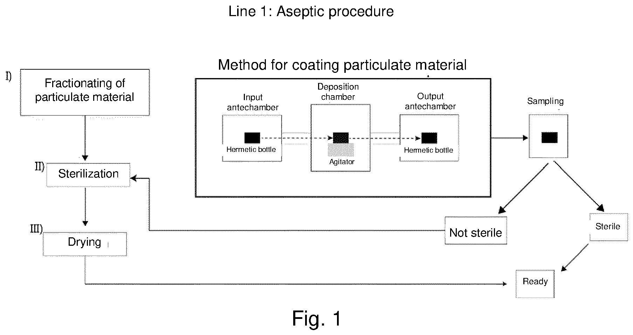

Fig. 1 shows the process flow and sputtering equipment.

### Detailed Description of the Invention

The main advantages of the inventive equipment and sputtering method are:
- Manufacturing of a sterile product;
- Efficient process, since one batch can begin undergoing the process before the other is completed (semi-continuous production);
- Precision of the coated (deposited) layer due to controlling the agitation frequency, powder mass ratio, cup opening area, oscillation frequency, and chemical interaction between the powder and the material to be deposited. The oscillation frequency and the powder mass are parameters that exhibit an inverse relationship - that is, the greater the powder mass, the smaller the oscillation frequency must be in order for the coat to be homogeneous. Another relationship is cup opening area and deposition time; it was observed that the larger the area of the cup, the shorter the deposition time to obtain a compact and homogeneous coat. The chemical interaction between the powder and the material that is to be deposited may also influence the homogeneity of the coat. In this case, the greater the chemical interaction between the powder and the deposited material, the more compact and homogeneous the deposited coat will be.
- Reduction of human resources, since it is a semi-automated method;

The equipment was designed to manufacture sterile coated particles (through deposition), preferably through sputtering, especially for use in the medical field, such as in intradermal fillers.

Devices such as antechambers have been developed for this which eliminate the contact of the particulate material with the external environment. Such antechambers work in a semi-automated system.

Another device that comprises the equipment is the deposition gun, which has the purpose of spraying the material on the target.

The equipment also operates semi-continuously, meaning that a production batch can be started before the previous one is finished. That is, when the coated material travels to the outlet antechamber, another batch can be inserted into the inlet antechamber.

The equipment for cathodic deposition comprises:
a) inlet antechamber;
b) deposition chamber;
c) outlet antechamber.

The automated process of the opening of the hermetic bottle and conveyance from the inlet antechamber to the deposition chamber takes place in the inlet antechamber through the use of a pressure difference between the chambers through a retractable tube made of inert material such as 316L stainless steel, for example.

In particular, the deposition chamber comprises at least one spray gun.

The deposition chamber also comprises an agitator.

The automated method of conveyance from the deposition chamber to the outlet antechamber and hermetic closure of the bottle takes place in the outlet antechamber through the use of a pressure difference between the chambers through a retractable tube made of inert material such as 316L stainless steel, for example.

The first step of the method begins with the fractionation of the material into the desired portions.

After this step, the particulate material is sterilized. This operation can be carried out with either moist or dry heat.

The next step is to check whether the particulate matter is completely dry (moisture content less than 0.1%), and if it is still wet, it must be dried in a vacuum oven. The particulate material is sealed in a hermetic container.

The next step comprises loading the particulate material into the hermetic container in the inlet antechamber.

After loading and closing the inlet antechamber, at least one vacuum pump is connected thereto which will reduce the pressure by up to 10⁻⁸ mbar.

Afterward, the hermetic bottle is opened through an automated mechanism, and the pressure is maintained at up to 10⁻⁸ mbar. Immediately, in the deposition chamber, a gate valve is opened in order to allow access between the inlet antechamber and the deposition chamber. Through an automated system, the particulate material is poured from the hermetic container into the stirring cup through a retractable tube within the deposition chamber.

After the transfer, the gate valve is closed, thereby eliminating access between the chambers.

Subsequently, there is the possibility of starting a new batch through the introduction of a new hermetic container comprising sterile and dry particulate material into the inlet antechamber.

The spraying of the particulate material takes place in the next chamber, the deposition chamber. The base pressure must be maintained between 10⁻⁴ mbar and 10⁻⁹ mbar, and the working pressure between 10⁻¹ and 10⁻³ mbar. Prior to the spraying process, the spray guns undergo a cleaning process (pre-sputtering) in which the surfaces of the targets are cleaned in order to remove the oxide coat and leave the material to be sprayed with a high degree of purity. The spray guns are protected by screens during this cleaning process so that there is no contamination in the particulate material.

After the cleaning process, the power parameters must be set between 5 and 1000 W and the process time between 1 and 8 h, according to requirements.

The desired final thickness conditions are controlled according to the relationship between time and power: This relationship established as a deposition rate can vary from 0.015 nm.s⁻¹ to 1.4 nm.s⁻¹.

As soon as the deposition time between 1 and 8 hours has lapsed, the voltage, agitation, and gas injection must be turned off. At this point, the particulate matter is already coated.

After spraying, the coated particulate material is transferred from the stirring cup (deposition chamber) to a hermetic container (outlet chamber). For this purpose, the pressure of the outlet chamber must be maintained between 10⁻² and 10⁻⁸ mbar. In the deposition chamber, a gate valve is opened in order to allow access between the outlet chamber and the deposition chamber. Through an automated system, the coated particulate material is poured from the stirring cup into the container through a retractable tube within the deposition chamber.

After the transfer, the gate valve is closed, thereby eliminating access between the chambers.

The outlet chamber is filled with inert gas (which can be nitrogen or argon), which prevents contamination of the coated particulate material, whereupon the container is hermetically closed.

### Example 1. Preferred embodiment

One embodiment of the method for sputtering titanium on polymethyl methacrylate (PMMA) microparticles comprises the steps of:
1. Fractionating PMMA microparticles in a class II safety cabinet inside containers from 0.1 kg to 2 kg, depending on the batch requirement;
2. sterilizing the PMMA microparticles (can be by moist heat or dry heat):
   2.1. sterilization by moist heat is performed by placing the container with the PMMA microparticles in an autoclave and maintaining the temperature, pressure, and time parameters at 121 °C, 1.5 atm, and 15 min;
   2.2. sterilization by dry heat is performed by placing the container with the PMMA microparticles in a furnace and maintaining the parameters for temperature at 160°, atmospheric pressure, and time at 2 hours;
   2.3. the moisture content by percent of the PMMA microparticles is checked; if it is equal to or greater than 0.1%, the container containing the PMMA microparticles must be dried in a vacuum oven for a period of 4 h to 12 h at a temperature between 100 °C and 150 °C and a pressure of 10⁻¹ mbar, preferably for 4 h at 100 °C and a pressure of 10⁻¹ mbar;
   2.4. in an inert gas atmosphere (argon or nitrogen gas atmosphere), the container containing the PMMA microspheres is hermetically closed;
3. the container containing particulate material is loaded into the inlet antechamber, hermetically closed;
   3.1. pressure is maintained at 10⁻² mbar;
   3.2. the hermetically sealed bottle is opened by automation;
   3.3. the deposition chamber is opened, and the PMMA microparticles contained in the container are transferred to the stirring cup by automation;
   3.4. the deposition chamber is closed;
4. coating (depositing) the PMMA microparticles;
   4.1. the deposition chamber is maintained at a preferred base pressure of 10⁻⁴ mbar to 10⁻⁶ mbar;
   4.2. cleaning of the deposition guns:
      4.2.1 guns protected with screens;
      4.2.2 the stirrer is switched on at a frequency preferably between 10 to 60 Hz;
      4.2.3. the gas that is to be ionized in order to carry out the spraying of the target material is introduced until the preferred pressure of 10 ⁻³ mbar to 10 ⁻¹ mbar is reached;
      4.2.4. the voltage sources are switched on to the preferred power of 5-300 W for the preferred time of up to 15 min;
      4.2.5. after the cleaning process, the screens are removed from the guns;
      4.2.6. the voltage is maintained between 5 and 1000 W, preferably between 40 and 120 W, for a period of 0.5 to 6 hours, preferably from 5 to 6 hours;
      4.2.7. voltage switched off, agitation, and gas injection. At this point, the PMMA microspheres are coated (deposited).
5. Storage of PMMA microspheres, coated (deposited) with nanometric coats of metals:
   5.1. Transfer the coated (deposited) PMMA microspheres from the stirring cup inside the deposition chamber to the outlet antechamber. Method carried out by automation comprising the steps of:
   5.1.1. inserting the container with hermetic closure into the outlet antechamber;
   5.1.2. lowering and maintaining the outlet antechamber pressure at 10⁻² mbar;
   5.1.3. the deposition chamber is opened, and the PMMA microparticles contained in the stirring cup are transferred to the container with hermetic closure by automation;
   5.1.4. the deposition chamber is closed;
   5.1.5. the outlet antechamber is filled with inert gas, which can preferably be argon or nitrogen;
   5.1.6. the container containing the PMMA microspheres coated (deposited) with a nanometric coat of metal is hermetically closed.
6. Sample control:
   6.1. Under a laminar flow hood, remove a sample of microspheres from the hermetic container in order to carry out the physical-chemical and microbiological control;
   6.2. if the sample is contaminated, the sterilization operation must be repeated according to step 3, preferably 3.1;
   6.3. after this step, the container is hermetically closed.

## Claims

1. Equipment for depositing sprayed materials on particulate material, **characterized in that** it comprises:
a) inlet antechamber;
b) deposition chamber;
c) outlet antechamber.

2. The equipment as set forth in claim 1, **characterized in that** it comprises at least one spray gun in the deposition chamber.

3. The equipment as set forth in claim 1, **characterized in that** it comprises an agitator in the deposition chamber.

4. The equipment as set forth in claim 1, **characterized in that** it comprises an automated method for opening the hermetic container in the inlet antechamber.

5. The equipment as set forth in claim 1, **characterized in that** it comprises an automated method for conveying particulate material from the inlet antechamber to the deposition chamber.

6. The equipment as set forth in claim 1, **characterized in that** it comprises an automated method for conveying coated particulate material from the deposition chamber to the outlet antechamber.

7. The equipment as set forth in claim 1, **characterized in that** it comprises an automated method for closing the hermetic container in the outlet antechamber.

8. The equipment as set forth in claims 1, 5, and 6, **characterized in that** it comprises at least one retractable tube for the conveyance of particulate material from the inlet antechamber to the deposition chamber and from the deposition chamber to the outlet antechamber, preferably made of stainless steel.

9. A method for depositing sprayed materials on particulate material, **characterized in that** it comprises:
a) receiving the particulate materials;
b) fractionating the particulate materials;
c) sterilizing the particulate materials;
d) drying the particulate materials;
e) loading the particulate materials into the deposition antechamber;
f) coating (depositing) the particulate materials in the deposition chamber;
g) storing, checking, and sterilizing the particulate materials.

10. The method for depositing sprayed materials on particulate material as set forth in claim 9, **characterized in that** the sterilization is by wet or dry heat.

11. The method for depositing sprayed materials on particulate material as set forth in claim 9, **characterized in that** the loading in the inlet antechamber is carried out manually, where the particulate material is preferably located inside a hermetically closed container.

12. The method for depositing sprayed materials on particulate material as set forth in claim 9, **characterized in that** the opening of the hermetic container in the inlet antechamber is preferably carried out by automation.

13. The method for depositing sprayed materials on particulate material as set forth in claim 9, **characterized by** the automated transport of the particulate material from the inlet antechamber to the deposition chamber.

14. The method for depositing powdered materials on particulate material as set forth in claim 9, **characterized by** the transport of the particulate material from the inlet antechamber to the deposition chamber, preferably through a retractable tube, from the hermetic container to the stirring cup.

15. The method for depositing sprayed materials on particulate material as set forth in claim 9, **characterized in that** the step of coating (deposition) the particulate materials comprises the step of cleaning the guns.

16. The method for depositing sprayed materials on particulate material as set forth in claims 9 and 10, wherein the guns must be covered by a screen for cleaning.

17. The method for depositing sprayed materials on particulate material as set forth in claims 9 and 10, wherein the cleaning must be carried out at a preferred frequency of 10 to 60 Hz, at a preferred pressure of 10⁻² to 10⁻¹ mbar, for preferred time of up to 15 min, and at a preferred voltage of 5 to 300 W.

18. The method for depositing sprayed materials on particulate material as set forth in claim 9, wherein the coating step must be carried out at a preferred frequency of 5 to 35 Hz, at a preferred pressure of 10⁻³ to 10⁻¹ mbar, for a time of 0.5 to 6h, and at a voltage of 5 to 1000 W.

19. The method for depositing sprayed materials on particulate material as set forth in claim 9, wherein the coating step (deposition) should be preferably carried out within a period of 5 to 6 hours and at a voltage of 40 to 120 W.

20. The method for depositing sprayed materials on particulate material as set forth in claim 9, **characterized in that** the deposition step ends with the automated transport of the coated (deposited) particulate material from the deposition chamber to the outlet antechamber.

21. The method for depositing powdered materials on particulate material as set forth in claim 9, **characterized in that** the coated (deposited) particulate material is preferably transported through a retractable tube from the stirring cup to the container with hermetic closure.

22. The method for depositing sprayed materials on particulate material as set forth in claim 9, **characterized in that** the closing of the hermetic container in the outlet antechamber is carried out preferably by automation.

23. The method for depositing sprayed materials on particulate material as set forth in claim 9, **characterized in that** the opening of the hermetic container in the inlet antechamber is preferably carried out by automation.

24. The method for depositing powdered materials on particulate material as set forth in claim 9, **characterized in that** a sample of the coated (deposited) particulate material is removed in order to perform quality control.

25. The method for depositing sprayed materials on particulate material as set forth in claim 9, **characterized by** the sterilization and drying of the coated (deposited) particulate material.

26. The method for depositing sprayed materials on particulate material as set forth in claim 9, **characterized in that** the particulate material is preferably polymethyl methacrylate (PMMA), preferably of micrometric size.

27. The method for depositing sprayed materials on particulate material as set forth in claim 9, **characterized in that** the sprayed materials are preferably Ti, Ag, or Au.

28. The method for depositing sprayed materials on particulate material as set forth in claim 9, **characterized in that** it is a semi-continuous process.

29. The method for depositing sprayed materials on particulate material as set forth in claim 9, **characterized in that** the automation is controlled through the deposition parameters via automation of voltage sources, opening and closing of valves, gas injection, positioning of the hermetic cup, vacuum control and measurement, without limitation thereto.
